# EUROPEAN PATENT SPECIFICATION

(11) **EP 3 424 555 B1**
(45) Date of publication and mention of the grant of the patent: **29.06.2022**
(21) Application number: 18020272.3
(22) Date of filing: 21.06.2018
(51) Int. Cl.: A61M 39/22, A61M 39/24

(54) **VALVE DEVICE AND USE OF SUCH VALVE DEVICE AS A BACK CHECK VALVE IN AN INTRAVENOUS SYSTEM**
VENTILVORRICHTUNG UND VERWENDUNG SOLCH EINER VENTILVORRICHTUNG ALS RÜCKSCHLAGVENTIL IN EINEM INTRAVENÖSEN SYSTEM
DISPOSITIF DE SOUPAPE ET UTILISATION D'UN TEL DISPOSITIF DE SOUPAPE COMME SOUPAPE ANTI-SIPHON DANS UN SYSTÈME INTRAVEINEUX

(30) Priority: 22.06.2017 SE 1750810
(43) Date of publication of application: 09.01.2019
(73) Proprietor: CYTO365 AB, 263 65 Viken (SE)
(72) Inventor: TÖRNBLOM, Micael, 26365 Viken (SE)
(74) Representative: AWA Sweden AB

(56) References cited:
- WO-A1-2005/107847
- WO-A1-2013/182856
- WO-A1-2016/132093
- US-A- 2 538 662
- US-A1- 2007 272 311

## Description

### Technical field

The present invention refers to a valve device and the use of such valve device as a back check valve in an intravenous system (IV-system) used for infusion therapy, IV-therapy or to administer drugs to the patients' vascular system.

### Technical background

An intravenous system, also known as an IV-system, is a system where fluid flows from high pressure to low pressure. Typically, an IV-system consists of an infusion container with fluid located at a head height spatially located higher than the patient. The infusion container is connected to the patient via an infusion tubing and a vascular access device (VAD), such as a catheter. The IV -system should be an air free system and free from air bubbles to avoid air getting in to the patients' bloodstream with a potential risk of air embolism to occur. Therefor a drip chamber is normally placed either directly below the infusion container to catch any air bubbles in the system, or placed further downstream in a position often referred to as an "inline placement".

When administrating drugs to a patient via an IV -system, a back check valve may be found in various positions in the IV -system setup.

A back check valve may be used together with a VAD. The back check valve is often placed in close proximity of the VAD, to avoid backflow from the patients' bloodstream up through the catheter tip. This backflow may be a cause of e.g. a sudden rise of the patients' blood pressure, by a sudden movement of the patient or if the patient would cough. This sudden backflow is also called reflux and a back check valve may be used for the purpose to restrict flow in the upstream direction and thereby prevent back flow. A back check valve used for this purpose may be called an anti-reflux valve.

Often there is a need to gain access to the patients' bloodstream to aspirate blood by connecting a syringe to the VAD or to an access port located in close proximity of the VAD. The anti-reflux valve does however hinder the possibility to aspirate since the aspiration procedure requires a flow in the upstream direction. Accordingly, the anti-reflux valve must either be dismantled by opening the IV-system, or not be placed in the IV-system from the beginning. Alternatively, the anti-reflux valve may be positioned further apart from the VAD with an access port being arranged between the anti-reflux valve and the VAD.

An access port placed in close proximity of a VAD without any anti-reflux valve between the VAD and the access port may in the event of a back flow result in blood spillage at the access port, but also that blood is entering e.g. the catheter tip. This may in the worst case result in complication with infections, also known as Catheter Related Blood Stream Infections, CRBSI.

When connecting to an access port with a needle free connector e.g. with a Male Luer, this will often result in a positive pressure upon connection and a negative pressure upon disconnection. This negative pressure may also cause backflow of blood in the catheter tip with same risk of CRBSI.

When backflow occurs to the catheter tip there is a risk of clotting of the VAD. Clotting of the VAD is common over time especially since the VAD may be in situ for an extended period of time, normally ranging from 12h up to 96h. Clotting may however be reduced with a back check valve. To maintain patency, i.e. the degree of openness of the IV -system and hence prevent blockage, an anticoagulant may be used to flush the VAD. With present innovation, the need of using anticoagulant to maintain patency may be reduced, which is both time saving, and cost saving and the associated risk with usage of an anticoagulant drug is thereby also reduced.

A back check valve may also be used to prevent two or more drugs from intermixing when connected to the same IV-system. An IV-system may contain a main infusion tubing, IV tubing, also called main line or sometimes also referred to as a primary infusion tubing or primary line. In many cases one or more secondary infusion tubings, also referred to as secondary lines, are connected to the main line via access ports such as an Y-site, a stopcock or a manifold and sometimes a combination of the above and may be referred simply to an infusion set, IV-set. It is often a wish to separate the different lines from each other so that the respective drugs do not, due to pressure differences, back track upstream towards another infusion container and mix. By placing a back check valve on each IV tubing, the flow is restricted to one direction only.

There are many different types of drugs that may be connected to an IV - system and typically they should be separated with a neutral fluid such as Saline. If drugs are mixed, there could be a risk of drug incompatibility to occur. This is an unknown state of the drug where the drug may take a different character than intended, where the drug effect may be increased or reduced, or provide a completely different effect than intended. Adverse events due to drug incompatibility are costly and may involve hospital days and even intensive care.

If one of the secondary drugs is mixed with the neutral fluid it doesn't necessarily mean that drug incompatibility occurs in the neutral container, but the supposed neutral container will no longer be neutral, and may risk causing drug incompatibility when used as a separator of incompatible drugs.

To avoid air embolism of the patient, the IV -system must be free of air before connected safely to the patient and be maintained free of air even when connecting a secondary infusion to the main line. A drip chamber is typically used to assure that the main line, which is connected to the VAD, is free of air. As mentioned above it is important that the neutral fluid of the neutral container remains neutral. To safeguard this, a back check valve should be arranged below the neutral container. However, if there is a drip chamber arranged on the same infusion tubing below the back check valve, the back check valve is often omitted because there might be a need of refilling the drip chamber in case of the drip chamber runs empty. The refilling is made by squeezing the drip chamber to build up pressure that forces air upwards to the container and then creates an under pressure upon release. The drip chamber is then refilled with fluid from the container. If there is a back check valve arranged above the drip chamber, this refilling procedure would not be possible and the whole IV-system, and sometimes with all connected secondary infusions, must be discarded. It is therefore not uncommon to see an IV -system setup without a back check valve between the neutral chamber and the drip chamber.

When connecting secondary infusions to the main line and if the main line lacks a drip chamber below the access ports, the secondary infusions must be prefilled with fluid and contain no air. If a back-priming method is used, the secondary line to be connected does not need to be air-free. To back-prime a secondary set, fluid is used from the infusion container on the main line to fill the secondary line by holding the secondary line at a head height lower than the infusion container on the main line. This back-prime method is widely used in specifically the U.S. but cannot be achieved if there is a back check valve on the secondary infusion line.

US 2016/0089492A1 discloses a valve that is a back check valve that may be manipulated to allow fluid in the normally restricted direction. However, the document uses a complex bulky and flexible structure located inside a flexible housing inside the intended fluid path. If this valve is used for blood administration or blood aspiration there is a risk of blood clotting within the housing due to the flexible structure that is located in the fluid path. Furthermore, this house needs to be large enough to house the radially extending flexible structure. The large housing volume is disadvantageous since it is a reservoir in itself. All dead space needs to be carefully primed before use, and especially if used without a drip chamber downstream towards the patient, and must also be carefully flushed after use when administrating drugs or blood and/or aspirating blood to thereby avoid bacteria colonization within the valve.

### Summary

One object of the invention is to provide a valve device acting as a back check valve that may be manipulated to be open and allow fluid in the normally restricted direction.

It is an object of the invention that the valve device should be able to be used where it is normally needed but today not considered possible due to the need of aspirating close to a VAD or when refilling drip chamber on the main line, or when back-priming secondary lines.

Another object of the invention is to provide a valve device in the form of a back check valve that reduces the risk of CRBSI.

These and other objects are solved by a valve device comprising a housing comprising a passage extending between a proximal and a distal end of the housing; a plunger arranged to extend at least partly inside the passage of the housing; a valve sealing member arranged in the passage, the valve sealing member together with the plunger being configured to control a fluid flow through the passage; a flexible tubing having a distal end fixedly arranged to the housing, thereby forming a first fixing portion of the flexible tubing; wherein the plunger having a proximal end being arranged to the flexible tubing, thereby forming a second fixing portion of the flexible tubing; and a distal end configured to selectively act on the valve sealing member for selectively setting the valve device from an open state or a closed state to a forced open state; wherein the valve device allowing a fluid flow in a downstream direction in the open state from the proximal end of the housing to the distal end of the housing, and restricting a fluid flow in an upstream direction in the closed state from the distal end of the housing to the proximal end of the housing; and wherein the valve device upon being selectively set in the forced open state, a fluid flow in the upstream direction from the distal end of the housing to the proximal end of the housing is allowed; and wherein the valve device is arranged to be selectively set from an open state or closed state to the forced open state by affecting the length of the flexible tubing as seen between its first fixing portion and its second fixing portion, thereby displacing the plunger relative the housing.

The distal end of the flexible tubing may be fixedly arranged to the proximal end of the housing.

The proximal end of the plunger may be fixedly arranged to the flexible tubing.

Accordingly, a valve device is provided that is intended to be arranged in a conduit and allowing a fluid flow through the valve device to be selectively controlled. The valve device acts as a back check valve which depending on the flow direction either allows a flow (open state) or restricts a flow (closed state). To overrule the function as a back check valve, the valve device may be selectively set to a forced open state. The setting of the valve device to its forced open state is made by affecting the length of the flexible tubing as seen between its first fixing portion and its second fixing portion. The change in length causes a longitudinal displacement of the plunger relative the housing and thereby the possibility for the plunger to interact with the valve sealing member is controlled. The valve device may be set to its different states by applying an external force to the valve device transverse its longitudinal extension. In its most simple form, the force may be applied by the operators fingers only by simply compressing or extracting the valve device in its longitudinal direction.

The plunger may be relatively rigid as seen in its longitudinal direction in relation to the flexible tubing that is flexible in its longitudinal direction.

The first fixing portion and the second fixing portion of the flexible tubing may be separated by an intermediate portion extending along the longitudinal extension of the flexible tubing, and the flexible tubing may along its intermediate portion be unfixed relative to the proximal part of the housing and the plunger respectively.

The plunger may comprise a through-going bore, an open proximal end, and a radially extending opening adjacent the distal end, whereby the radially extending opening allows the fluid flow past the plunger through said through-going bore.

The distal end of the plunger may be provided with a continuous or discontinuous circumferential edge. In case the valve sealing member instead being a ball, the distal end may be concave to better engage the ball.

In another embodiment, the plunger may comprise a through-going bore, an open proximal end, a closed distal end and a radially extending opening adjacent the closed distal end, whereby the radially extending opening allows the fluid flow past the plunger through said through-going bore. The closed distal end may serve as a valve seat since the plunger interacts with the valve sealing member.

The valve device may be arranged to be selectively set to the forced open state by applying a force to an exterior side of the flexible tubing and the housing respectively, thereby causing a longitudinal length modification of the flexible tubing as seen between its first fixing portion and its second fixing portion.

Since the force is applied from the exterior side of the valve device, the valve device may be kept very lean.

The valve device may further comprise an actuator, the actuator being arranged on an exterior side of the flexible tubing and on an exterior side of the housing, the actuator having a distal end and a proximal end, and the actuator engaging the housing by the distal end of the actuator and engaging on the exterior of the flexible tubing by a proximal end of the actuator, whereby application of a force to the actuator in a transverse direction causes a longitudinal length modification of the flexible tubing between the first fixing position and the second fixing position thereby setting the valve device to its forced open state.

In one embodiment, the valve device may further comprise an actuator, the actuator being arranged on an exterior side of the flexible tubing and on an exterior side of the housing, the actuator having a distal end and a proximal end, and the actuator engaging the housing by the distal end of the actuator and engaging the plunger by the proximal end of the actuator, whereby application of a force to the actuator in a transverse direction causes a longitudinal length modification of the flexible tubing between the first fixing position and the second fixing position thereby setting the valve device to its forced open state.

In one embodiment, the flexible valve sealing member may comprise a through-going opening, and wherein the distal end of the plunger is arranged to abut the valve sealing member when the valve device is set to the closed state; whereby a fluid flow in the downstream direction from the proximal end of the housing to the distal end of the housing is allowed by the fluid flow forcing the flexible valve sealing member to unseat the distal end of the plunger, thereby allowing a fluid flow through the through opening in the flexible valve sealing member, and a fluid flow in the upstream direction from the distal end of the housing to the proximal end of the housing is restricted by the fluid flow forcing the flexible valve sealing member to sealingly close-off the through-going opening in the flexible valve sealing member.

The closing-off may be provided by the distal end of the plunger being closed and having a cross-section larger than the cross-section of the through-going opening in the valve sealing member. It is also possible to provide the distal end of the plunger with e.g. a cone-shape or a spherical shape which at least partly is inserted into the opening in the valve sealing member.

In one embodiment, the flexible valve sealing member may comprise a through-going opening, and wherein the distal end of the plunger is arranged to abut the valve sealing member when the valve device is in the closed state; whereby a fluid flow in the downstream direction from the proximal end of the housing to the distal end of the housing is allowed by the fluid flow forcing the flexible valve sealing member to unseat the distal end of the plunger, thereby allowing a fluid flow through the through opening in the flexible valve sealing member, and a fluid flow in the upstream direction from the distal end of the housing to the proximal end of the housing is restricted by the fluid flow forcing the flexible valve sealing member to sealingly close-off the through-going opening in the flexible valve sealing member.

In one embodiment, the valve sealing member may be flexible and comprise a first portion and a second portion, the second portion encircling the first portion, the first portion forming a homogenous sealing surface and the second portion comprising a through-going opening, wherein when the valve device is set in its forced open position, the plunger is displaced relative to the housing to such extent that the first portion of the flexible sealing member is deflected by the distal end of the plunger acting thereon, thereby forming an interspace between the first portion of the flexible sealing member and the valve seat to thereby allow a fluid flow in an upstream direction through the through-going opening in the flexible sealing member.

The proximal end of the plunger may comprise a transversely extending projection, and wherein the proximal end of the plunger may be fixedly arranged to the flexible tubing at the second fixing portion by the transversely extending projection engaging the inner proximal wall portion of the flexible tubing.

The projection may be arranged to interact with an actuator which may be used to set the valve device to its forced open state.

According to another aspect the invention refers to the use of a valve device with any of the features given above as a back check valve in an intravenous system.

The passage in the housing may comprise a chamber and an inner wall of the chamber may comprise a valve seat encircling the passage, and the valve sealing member may be retained in the chamber to at least partly abut the valve seat when the valve device is in its closed state thereby providing a sealing between the valve sealing member and the valve seat.

Further objects and advantages of the present invention will be obvious to a person skilled in the art reading the detailed description given below describing different embodiments.

The valve sealing member may be flexible and comprise a first portion and a second portion, the second portion encircling the first portion, the first portion forming a homogenous sealing surface and the second portion comprising a through-going opening, wherein when the valve device is in its open position a fluid flow in the downstream direction from the proximal end of the housing to the distal end of the housing is allowed by the fluid flow forcing the first portion of the valve sealing member to unseat the valve seat, thereby allowing a fluid flow past the valve seat and through the through opening in the second portion of the flexible valve sealing member.

The valve sealing member may be homogenous with no through-going holes. One example of a homogenous valve sealing member is a ball.

The valve sealing member may be attached to the plunger.

### Brief description of the drawings

The invention will be described in detail with reference to the schematic drawings.
FIG. 1A discloses a valve device according to a first embodiment in an open state allowing a downstream fluid flow, or a closed state preventing upstream fluid.
FIG. 1B discloses a valve device according to the first embodiment set in a forced open state allowing an upstream fluid flow.
Fig. 1C discloses a sectional view A-A of the valve device according to the first embodiment in an open state allowing a downstream fluid flow.
Fig. 1D discloses a sectional view A-A of the valve device according to the first embodiment in a closed state preventing an upstream fluid flow.
Fig. 1E discloses a sectional view B-B of the valve device according to the first embodiment set to a forced open state allowing an upstream fluid flow.
Fig. 2A discloses a perspective view of the valve device according to the first embodiment.
Fig. 2B discloses a partially exploded view of the valve device according to the first embodiment.
Fig. 3A discloses a valve device according to a second embodiment in an open state allowing a downstream fluid flow, or a closed state preventing upstream fluid.
Fig. 3B discloses the valve device according to the second embodiment set in a forced open state.
Fig. 3C discloses a sectional view A-A of a valve device according to the second embodiment in an open state allowing a downstream fluid flow.
Fig. 3D discloses a sectional view A-A of a valve device according to a second embodiment in a closed state preventing upstream fluid flow.
Fig. 3E discloses a sectional view B-B of the valve device according to the second embodiment set to a forced open state allowing an upstream fluid flow.
Fig. 4 discloses a perspective view of one embodiment of an actuator in combination with a clamp function on a valve device according to the first embodiment.
Fig. 5 discloses a perspective view of one embodiment of an actuator with a locking device arranged on a valve device according to the second embodiment.
Fig. 6A discloses a valve device with a plunger integrated with an inlet on a valve device according to a third embodiment.
Fig. 6b discloses a sectional view A-A of a valve device with a plunger integrated with an inlet on a valve device according to a third embodiment.
Fig. 6C discloses a perspective view of a valve device according to the third embodiment.

### Detailed description

To facilitate the description, the terms transverse and longitudinal are used. The term "*longitudinal*" refers to the intended fluid flow through the valve device during normal operation. Also, the terms proximal and distal are used in order to better describe the geometrical interrelation between the different parts of the valve device. Further, the terms downstream and upstream are used to describe the flow. It is to be stressed that these extensions and directions do not provide any restrictions in terms of how the valve device is oriented when in use but are merely used to better describe the function. To avoid undue repetition, some reference numbers may be found only in one of the figures, where the parts are the same.

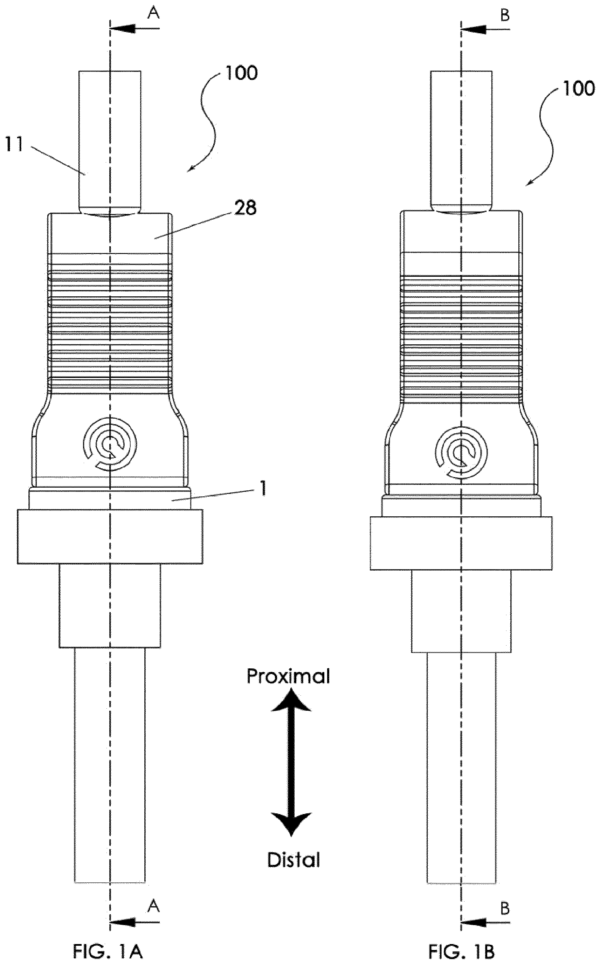

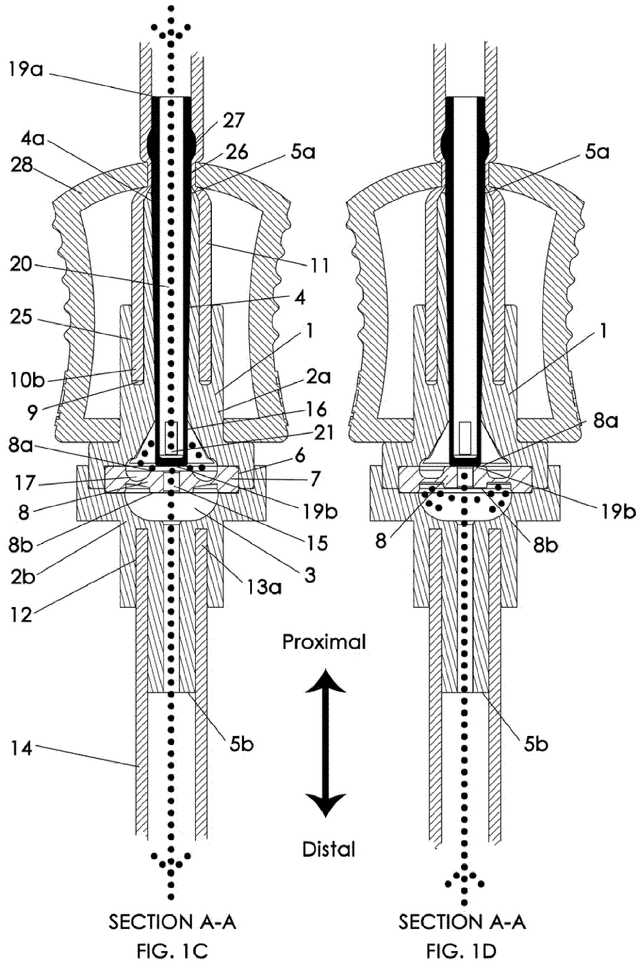

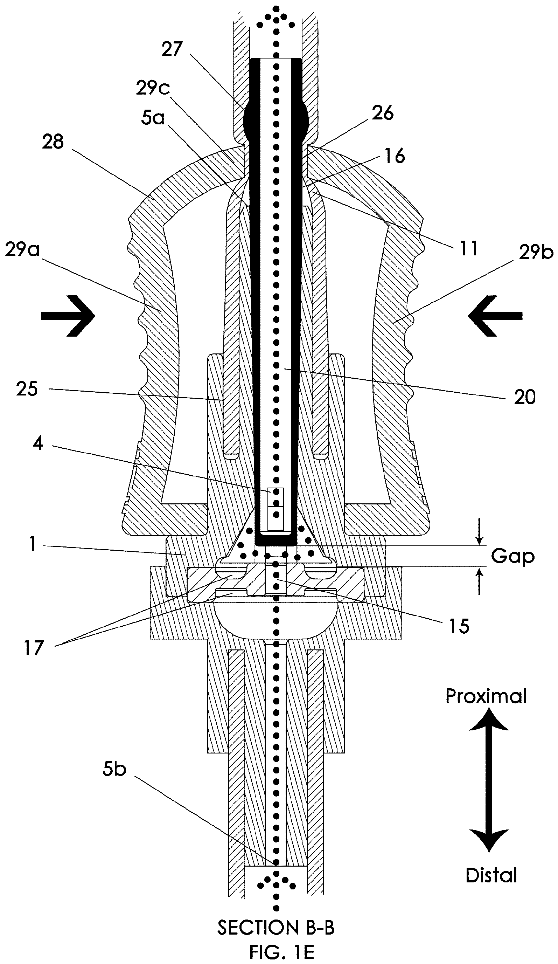

In Figs. 1A-1B, a first embodiment of the valve device 100 is disclosed. In Fig. 1A the valve 100 is in its non-active mode where section A-A will be explained in Figs. 1C-1D. In Fig. 1B the valve 100 is in its active mode where section B-B will be explained in Fig. 1E.

Now turning to Fig. 1C,1D the overall design of a valve device 100 according to a first embodiment is disclosed.

The valve device 100 comprises a housing 1. The housing 1 comprises a proximal part 2a and a distal part 2b. The two parts 2a, 2b are joined to form a closed housing 1 delimiting a chamber 3 and a through passage 4 extending between a proximal end 5a of the housing 1 and a distal end 5b of the housing 1. The chamber 3 comprises, as seen in the transverse direction, a recess 6. The recess 6 is configured to interact with an edge portion 7 of a valve sealing member 8 to prevent this from being displaced along the passage 4 in the longitudinal direction in the view of the passage 4.

The proximal part 2a of the housing 1 comprises a circumferential recess 9 configured to interact with a distal end 10b of a flexible tubing 11 to be discussed below.

The distal part 2b of the housing 1 comprises a circumferential recess 12 configured to interact with a proximal end 13a of a tubing 14 to be discussed below. The proximal and distal parts 2a, 2b of the housing 1 may be formed by e.g. an injection molded plastic material. The proximal and distal parts 2a, 2b of the housing 1 are joined to each other to form a fluid tight body.

The valve sealing member 8 is received in the chamber 3. The valve sealing member 8 is provided as a disc which is retained in the housing 1 by the edge portion 7 of the valve sealing member 8 being received in the recess 6 of the chamber 3.

The valve sealing member 8 comprises a central through-going opening 15. The through-going opening 15 is aligned with the longitudinal extension of a plunger 16 to be discussed below. More than one through-going opening 15 may be provided.

The valve sealing member 8 may be provided with a recess 17 which is arranged in the proximal and/or distal surface 8a, 8b of the valve sealing member 8. The recess 17 may be arranged in the circumferential direction of the valve sealing member 8 and facilitate deflection or bulging of the valve sealing member 8 in the longitudinal direction when subjected to an external force applied by the plunger 16 or by a fluid flow. The valve sealing member 8 may be provided by a flexible material, such as plastics or silicon.

The plunger 16 is arranged to extend at least partly inside the passage 4 of the housing 1. The plunger 16 is relatively rigid as seen in its longitudinal direction in relation to the flexible tubing to be discussed below. The plunger 16 has a proximal end 19a extending past the proximal end 5a of the housing 1 and a distal end 19b extending into the chamber 3 of the housing 1. The plunger 16 comprises a through-going bore 20, an open proximal end 19a, a closed distal end 19b and a radially extending opening 21 adjacent the distal end 19b. The closed distal end 19b of the plunger 16 may have more than one radially extending opening 21. The radially extending opening 21 allows a fluid flow past the plunger 16 through said through-going bore 20. The proximal end 19a of the plunger 16 may comprise a transversely extending projection 27 to be discussed below. The plunger 16 may have an outer cross section complementary to the cross section of the passage 4 in the housing 1. As the plunger 16 is moved relative the housing 1, the plunger 16 may be guided by an inner wall 4a of the passage 4.

The closed distal end 19b of the plunger 16 may have a cross-sectional area that exceeds the cross-sectional area of the through-going opening 15 in the valve sealing member 8. The plunger 16 may be provided by a rigid or semi-rigid material such as plastic material or even some metal or composite thereof to withstand compression when affecting the valve sealing member 8.

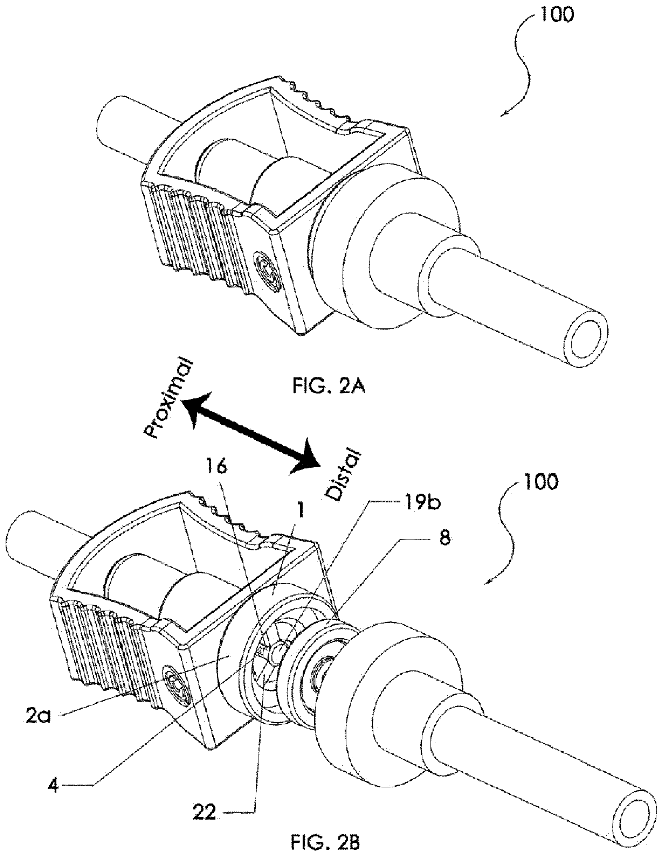

As is best seen in Fig. 2B disclosing a partially exploded view of the housing 1, the proximal part 2a of the housing 1 may comprise one or more retainers 22. The retainer 22 may be formed as radially extending spokes having an extension transverse the longitudinal extension of the passage 4. The retainer 22 comprises a central through-going opening 24 allowing the distal end 19b of the plunger 16 to extend there through. The retainer 22 prevents the valve sealing member 8 from being displaced or bulge in the upstream direction. Thereby the axial distance between the valve sealing member 8 and the closed distal end 19b of the plunger 16 will be well defined and the length of stroke of the plunger 16 required to set the valve device 100 to its forced open position may be well defined and is also reduced.

Now turning to Fig. 1C, the distal end 10b of the flexible tubing 11 is fixedly connected to the proximal part 2a of the housing 1, thereby forming a first fixing portion 25 of the flexible tubing 11. In the disclosed embodiment, the distal end 10b of the flexible tubing 11 is fixedly received in the circumferential recess 9 in the proximal part 2a of the housing 1. The fixing portion may be either on the external or internal side of the tubing 11 or at both sides.

The proximal end 19a of the plunger 16 is fixedly arranged to the flexible tubing 11 at a second fixing portion 26 of the plunger 16 engaging an inner proximal wall portion of the flexible tubing 11. Thus, the proximal end 19a of the plunger 16 is inserted into the flexible tubing 11. A radially extending projection 27 will aid the plunger to be fixed in relation to the tubing at the second fixing portion 26, which may be achieved by friction only. This results in a co-movement of the plunger 16 and of the tubing at the second fixing portion 26 when an external force from the outside of the tubing is exerted in the transverse or longitudinal direction at the projection 27. The first and second fixing portions 25, 26 are arranged at a distance from each other as seen in the longitudinal extension of the valve device 100. The first fixing portion 25 and the second fixing portion 26 of the flexible tubing are separated by an intermediate portion extending along the longitudinal extension of the plunger 16. The intermediate portion of the flexible tubing 11 is unfixed relative to the proximal part of the housing and the plunger respectively.

The fixation at the first fixing portion 25 may be made by e.g. bonding using an adhesive, chemical bonding or mechanical interlocking. The second fixing portion 26 may be achieved by friction only, adhesive, chemical bonding or mechanical interlocking.

It should be noted that the second fixing portion 26 may be a fixing portion selectively, resulting in a second fixing portion only when required e.g. when the plunger is activated to manipulate the sealing valve member, and selectively the plunger may be relatively unfixed from the tubing. This may be achieved if friction is used as a fixation method between plunger and tubing.

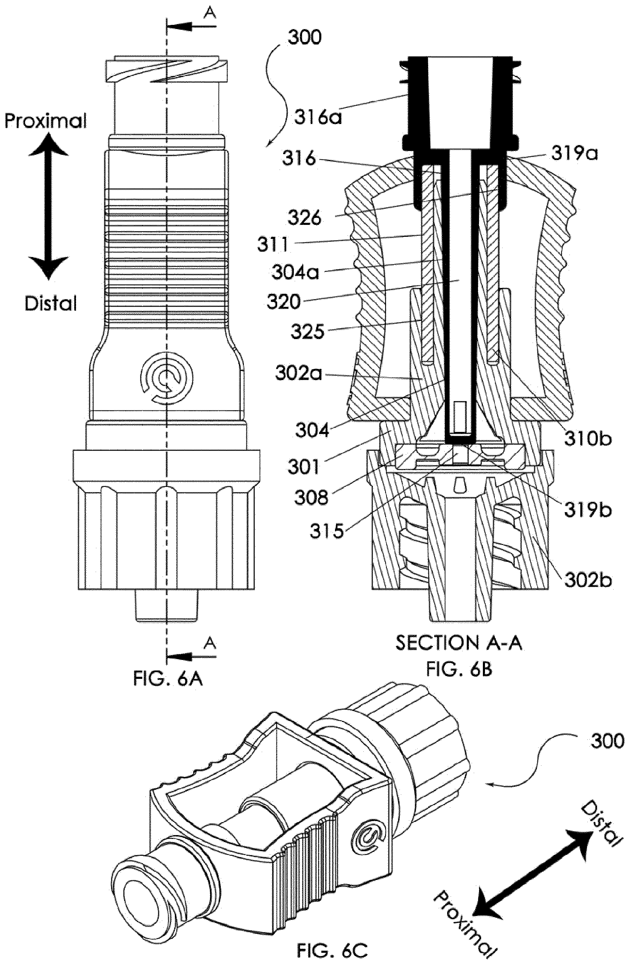

The distal end 5b of the housing 1 may be connected to a tubing 14. The tubing 14 may be flexible or rigid. In an alternative solution to the distal end 5b being connected to a tubing 14, the distal part 2b of the housing 1 may be formed as a standardized Luer connector of a male or a female type, see Fig. 6A-6C.

The valve device 100 comprises an actuator 28. The actuator 28 is arranged on an exterior side of the flexible tubing 11 and on an exterior side of the housing 1. The actuator 28 engages the proximal part 2a of the housing 1 and the second fixing portion 26 of the flexible tubing 11. It is to understood that the actuator 28 with remained function may be attached to either the distal part 2b of the housing 1 with e.g. bonding or mechanical attachment or to the proximal part 2a of the housing 1 without the need of a bonding since the proximal part 2a of the housing 1 will act as an anvil for the resulting longitudinal extraction force in the valves active state.

The actuator 28 may be formed as one unitary body by a flexible material such as plastic.

Now turning to Fig. 1E. When applying a compression force, see arrows, to the actuator 28 in a direction transverse the longitudinal extension of the flexible tubing 11 the compression causes an elongation of the flexible tubing 11 in the portion of the flexible tubing 11 that extends between the first and second fixing portions 25, 26. This elongation allows the valve device 100 to be manually operated to set the valve device 100 from a closed position to a forced open position.

In the following the operation of the valve device 100 will be described. The valve device 100 is intended to be arranged in a conduit allowing a fluid flow through the valve device 100 to be selectively controlled. The valve device 100 acts as a back check valve which depending on the flow direction either allows a flow (open state) in a downstream direction or restricts a flow (closed state) in an upstream direction. To overrule the function as a back check valve, the valve device 100 may be selectively set to a forced open state allowing a flow in an upstream direction

Now turning to Fig. 1C, the open state will be described. In the open state, the valve device 100 allows a fluid flow (dotted line) in a downstream direction from the proximal end 5a of the housing 1 to the distal end 5b of the housing 1. When supplying a fluid flow in the downstream direction, the fluid is allowed to flow through the flexible tubing 11 connected to the proximal end 2a of the housing 1. The fluid flows through the passage 4 along the plunger 16 and exits the same via the radially extending opening 21 in the plunger 16. The valve sealing member 8 has an inherent flexibility allowing the same to slightly deflect or bulge in a direction away from the distal end 19b of the plunger 16, thereby allowing the fluid to pass the valve sealing member 8 via its through-going opening 15 to thereby exit the valve device 100 via the distal part 2b of the housing 1.

Now turning to Fig. 1D, the closed state will be described. In the closed state, the valve device 100 restricts a fluid flow (dotted line) in an upstream direction from the distal end 5b of the housing 1 to the proximal end 5a of the housing 1. When supplying a fluid flow in the upstream direction, the fluid flow will act on the distal side 8b of the valve sealing member 8 and force the proximal end 8a of the valve sealing member 8 into a sealing engagement with the closed distal end 19b of the plunger 16. The fluid will thereby be prevented from passing the valve sealing member 8 and further into the proximal part 2a of the housing. Thus, the closed distal end 19b of the plunger 16 may be seen as a valve seat which sealingly engages and closes-off the through-going opening 15 in the valve sealing member 8.

Now turning to Fig. 1E, the forced open state will be described. In the forced open state, the valve device 100 allows a fluid flow (dotted line) in the upstream direction from the distal end 5b of the housing 1 to the proximal end 5a of the housing 1.

To set the valve device 100 to the forced open state, the length of the flexible tubing 11 as seen between its first fixing portion 25 and its second fixing portion 26 is affected, thereby causing the plunger 16 to be displaced relative to the housing 1. The length is affected by applying a compressing force to the opposing legs 29a, 29b of the actuator 28, thereby forcing the flexible legs 29a,29b towards each other, and thereby forcing the flexible leg 29c to deflect in a longitudinal direction in the view of the plunger. This will cause the plunger 16 to displace in its longitudinal direction in view of the housing 1 and cause a gap between the closed distal end 19b of the plunger 16 and the valve sealing member 8. Thereby the valve sealing member 8 is unseated and a free passage is formed through the through-going opening 15 in the valve sealing member 8 allowing an upstream fluid flow through the housing 1. This free passage is maintained as long as the actuator 28 is maintained in its activated state.

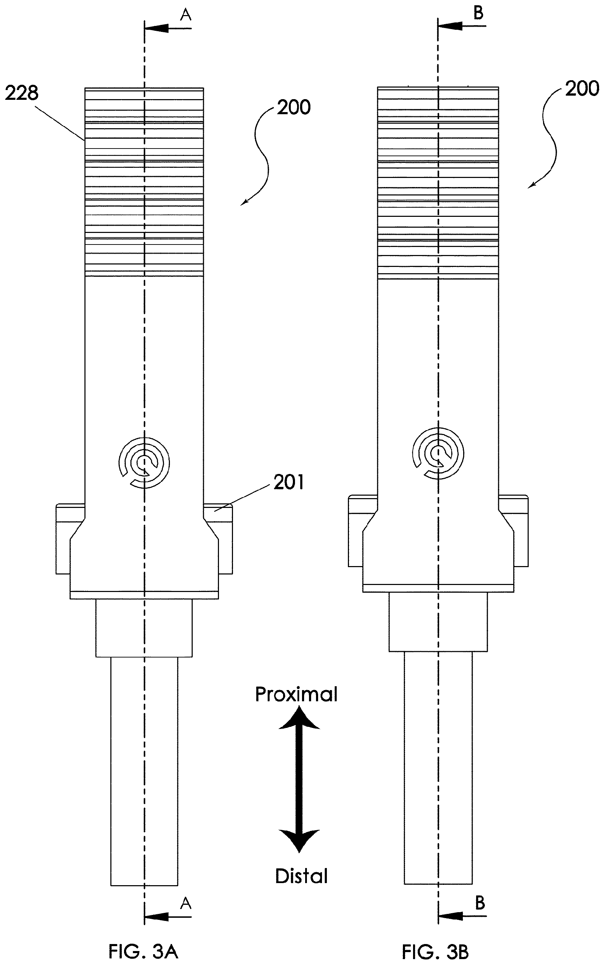

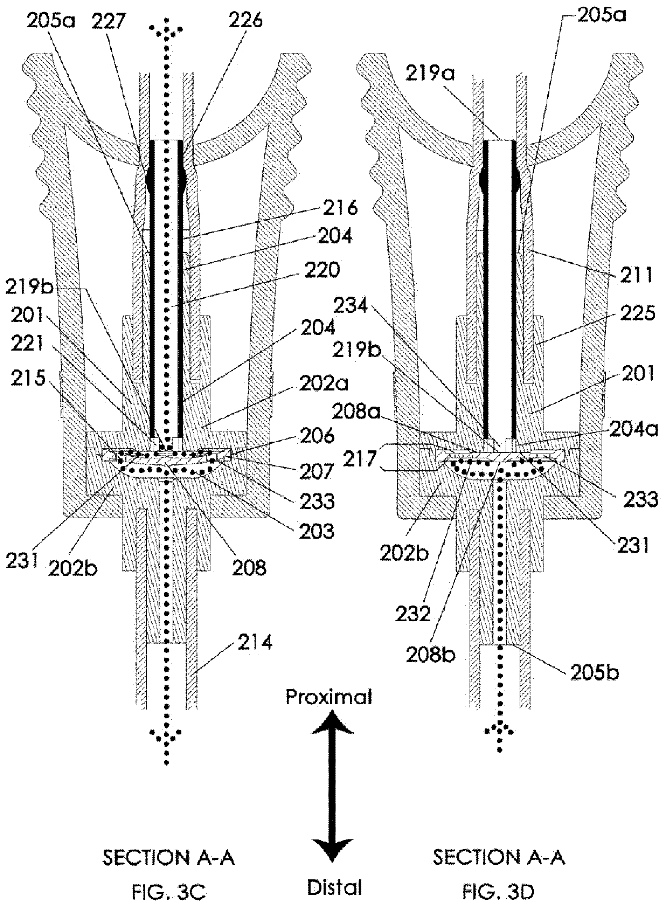

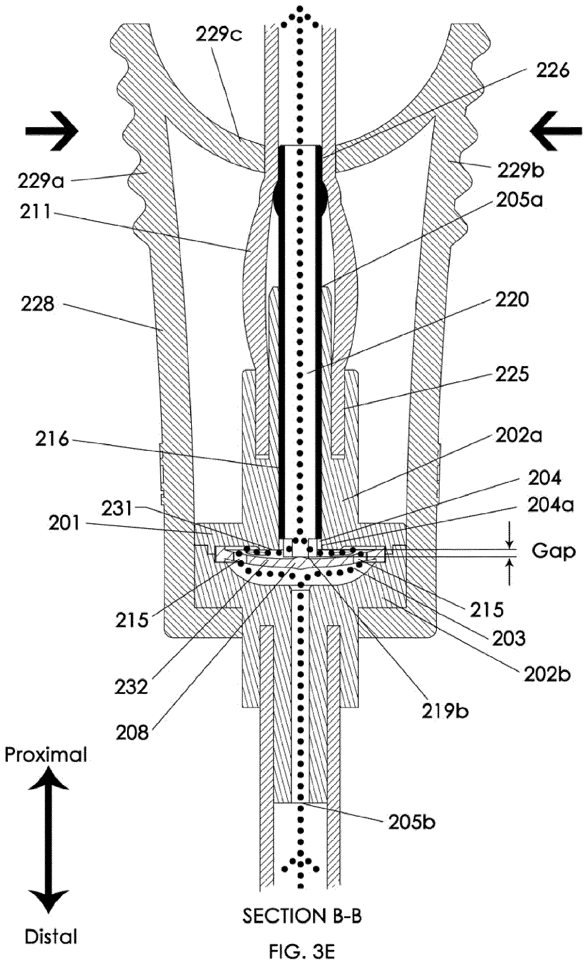

Now turning to Figs. 3A-3B, a second embodiment of the valve device 200 is disclosed. In Fig. 3A the valve 200 is in its non-active mode where section A-A will be explained in Figs. 3C-3D. In Fig. 3B the valve 200 is in its active mode where section B-B will be explained in Fig. 3E.

Now turning to Figs. 3A-3D.

The valve device 200 comprises a housing 201. The housing 201 differs from the housing 1 of the first embodiment in that the chamber 203 comprises a valve seat 231. The valve seat 231 is arranged in the proximal part 202a of the housing 201 and encircles the passage 204. The chamber 203 comprises, as seen in the transverse direction, a recess 206. The recess 206 encircles the valve seat 231 and is configured to interact with an edge portion 207 of a valve sealing member 208 to prevent it from being displaced in the longitudinal direction in the view of the passage 204. The valve sealing member 208 is retained in the recess 206 to at least partly abut the valve seat 231 when the valve device 200 is in its closed state, thereby providing a sealing between the valve sealing member 208a and the valve seat 231.

The valve sealing member 208 is provided as a flexible disc. The valve sealing member 208 comprises a first portion 232 and a second portion 233, the second portion 233 encircling the first portion 232. The first portion 232 comprises a homogenous sealing surface and the second portion 233 comprises a through-going opening 215. The through-going opening 215 is radially displaced in view of a longitudinal center axis of the plunger 216. It is to be understood that more than one through-going opening 215 may be provided. The through-going opening 215 is radially displaced to such extant that when the valve device 200 is in its closed state (to be discussed below) thereby providing a sealing between the valve sealing member surface 208a and the valve seat 231, no fluid is allowed to pass the through-going opening 215.

The valve sealing member 208 may be provided by a flexible material, such as plastics or silicon.

The plunger 216 has the overall same design as the plunger 16 in the first embodiment of the valve device 100. To avoid undue repetition, reference is made to the section above referring to the design of the plunger 16. The plunger 216 of the second embodiment differs in that it comprises a through-going bore 220, an open proximal end 219a, a distal end 219b and a radially extending opening 221 adjacent the distal end 219b.

The distal end 219b of the plunger 216 may be provided with two or more longitudinally extending fingers 234. The fingers 234 may between themselves form the radially extending opening 221. The fingers 234 provide the edge of the plunger 216 with a dis-continuous extension. In an alternative, not disclosed embodiment, the edge of the distal end 219b may be continuous and the radially extending openings 221 arranged at a small distance from the distal edge. In case of the valve sealing member 208 instead being a ball (not disclosed), the distal end 219b may be concave to better engage the ball.

The proximal end 202a of the housing 201 is connected to a flexible tubing 211 in a corresponding manner as has previously been described with reference to Figs. 1C-1E. The distal end 202b of the housing 201 is connected to a tubing 214 in a corresponding manner as has previously been described with reference to Figs. 1C-1E. To avoid undue repetition, reference is made to these sections. In an alternative solution to the distal part 202b of the housing being connected to a tubing 214, the distal part 2b of the housing 1 may be formed as a standardized Luer connector of a male or a female type, see Fig. 6A-6C.

Now turning to Fig. 3E. The valve device 200 comprises an actuator 228. The actuator 228 engages the distal part 202b of the housing 201 and a second fixing portion 226 of tubing 211. It is to be understood that the actuator 228 may be attached to either the proximal part 202a of the housing 201 with e.g. bonding or mechanical attachment or to the distal part 202b of the housing 201 without the need of a bonding since the distal part 202b of the housing 201 will act as an anvil for the resulting longitudinal compression force in the valves active state.

The actuator 228 may be formed as one unitary body by a flexible material such as plastic.

When applying a compression force indicated by the arrows in Fig. 3E, to the actuator 228 in a direction transverse the longitudinal extension of the flexible tubing 211, the compression causes a compression of the flexible tubing 211 in the portion of the flexible tubing 211 that extends between the first and second fixing portions 225, 226. This compression allows the valve device 200 to be manually operated to set the valve device 200 from a closed position to a forced open position.

In the following the operation of the valve device 200 will be described. The valve device 200 is intended to be arranged in a conduit allowing a fluid flow through the valve device 200 to be selectively controlled. The valve device 200 acts as a back check valve which depending on the flow direction that either allows a flow (open state) or restricts a flow (closed state). To overrule the function as a back check valve, the valve device 200 may be selectively set to a forced open state.

Now turning to Fig. 3C, the open state will be described. In the open state, the valve device 200 allows a fluid flow (dotted line) in a downstream direction from the proximal end 205a of the housing 201 to the distal end 205b of the housing 201. When supplying a fluid flow in the downstream direction, the fluid is allowed to flow through the flexible tubing 211 connected to the proximal end 205a of the housing 201. The fluid flows through the passage 204 along the plunger 216 and exits the same via the radially extending opening 221. The valve sealing member 208 has a flexibility allowing the same to slightly deflect or bulge in a direction away from the distal end 219b of the plunger 216. As a result of the deflection or bulging, the first portion 232 of the valve sealing member 208 unseats the valve seat 231 in the housing 201. The fluid is thereby allowed to pass the valve sealing member 208 via the through-going opening 215 in the second portion 233 to thereby exit the valve device 200 via the distal part 202b of the housing 201.

Now turning to Fig. 3D, the closed state will be described. In the closed state, the valve device 200 restricts a fluid flow (dotted line) in an upstream direction from the distal end 205b of the housing 201 to the proximal end 205a of the housing 201. When supplying a fluid flow in the upstream direction, the fluid will act on the distal side 208b of the valve sealing member 208 and force the same into a sealing engagement between the first portion 232 of the valve sealing member 208 and the valve seat 231. The fluid will thereby be prevented from passing the valve sealing member 208 and hence pass the valve device 200.

Now turning to Fig. 3E, the forced open state will be described. In the forced open state, the valve device 200 allows a fluid flow (dotted line) in the upstream direction from the distal end 205b of the housing 201 to the proximal end 205a of the housing 201.

To set the valve device 200 to the forced open state, the length of the flexible tubing 211 as seen between its first fixing portion 225 and its second fixing portion 226 is affected by causing a reduction of the length. Thereby the plunger 216 is displaced relative the housing 201. The length is affected by applying a pushing force on the actuator 228 as indicated by the arrows in Fig. 3E, thereby forcing the legs 229a,229b towards each other resulting in a compression of the actuator 228 in a transverse direction and a compression of the flexible leg 229c, resulting in a compression of the flexible tubing 211 in at least the portion of the flexible tubing 211 that extends between the second fixing portion 226 and the first fixing portion 225. The compression force on the actuator 228 and the enabled compression of the tubing 211 will cause the plunger 216 to displace relative to the housing 201 to such extent that the first portion 232 of the flexible sealing member 208 is deflected by the distal end 219b of the plunger 216 acting thereon. The deflection causes formation of a gap between the first portion 232 of the flexible sealing member 208 and the valve seat 231. Thus, a free passage is formed through the through-going opening 215 or openings in the valve sealing member 208 allowing an upstream fluid flow through the housing 201. This free passage is maintained as long as the plunger 216 is maintained in its activated state.

The skilled person will understand that as an alternative (not disclosed) to the valve sealing member 208 of the second embodiment of the valve device 200 having a flexible disk, the valve sealing member 208 may be formed as a ball or a cone that is received in the chamber 203 and which is arranged to either seat or unseat the valve seat 231 in the chamber 203 depending on the flow direction.

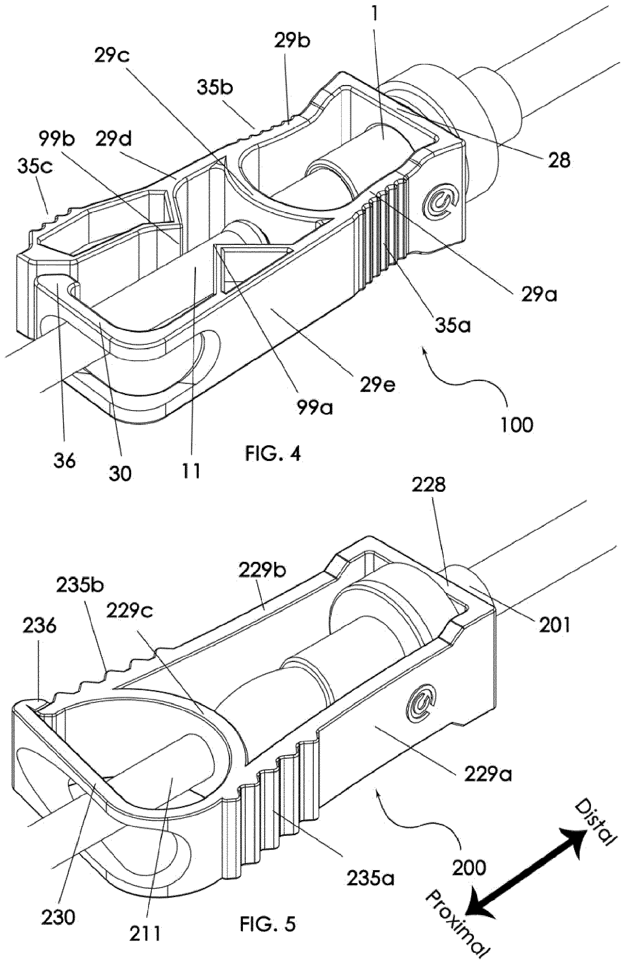

Now turning to Fig. 4, showing the first embodiment of the valve device 100 in a non-activated state with a different actuator 28. The actuator differs from the actuator previously discussed in view of the first embodiment in that it comprises a clamp, also known as a pinch clamp, to control fluid flow to enter the valve house 1.

The clamp comprises two opposing legs 29d, 29e forming extensions of the opposing legs 29a, 29b. The two opposing legs 29d, 29e comprises studs 99a, 99b adapted to face the flexible tubing 11 that extends between and along the legs. One of the opposing legs 29d comprises a grip surface 35c arranged in a proximal end thereof.

The clamp works as any ordinary pinch clamp to either close off or open the flow by letting the operator apply a pushing force on the grip surface 35c and the opposite leg 29e with e.g. finger and thumb, resulting in that the two studs 99a, 99b squeezes on the flexible tubing 11 and close off the fluid flow path. It should be noted that when the operator applies a pushing force at grip surfaces 35a, 35b of the opposing legs 29a, 29b, the flexible opposing legs 29a, 29b will compress inwards towards each other while the extended portion of the same legs 29d, 29e will extend outwards away from each other. Thereby the studs 99a,99b spaces apart allowing a fluid flow though the valve device 100. By this innovative design the clamp is not only adjacent placed close to the valve device 100 but also integrated. If a completely closed off fluid is needed this is achievable by the clamp and may be maintained to a closed off state with a locking mechanism 30 comprising a hook 36.

Now turning to Fig. 5 showing the second embodiment of the valve device 200 in an active state with a different actuator 228 now with a locking means 230 is disclosed. The actuator 228 is arranged on an exterior side of the flexible tubing 211 and on an exterior side of the housing 201. The actuator 228 comprises two opposing, longitudinally extending, flexible legs 229a, 229b. The actuator 228 may be formed as one unitary body of a plastic material. The two opposing legs 229a, 229b, comprise grip surfaces 235a, 235b, indicating to an operator where to apply the compression force. The actuator 228 comprises a locking means 230 in the form of a hook 236 that is operable between an open position and a locked position. The locking means 230 is disclosed in its locked and active position and allows maintaining of the applied force as long as the actuator 228 is set to its locked position.

Both Fig. 4-5 may have further combinations of functions and only one state is shown and the other states are instead referred to previous explanations of Figs. 1-3.

Now turning to Fig. 6A-C, showing an embodiment 300 of a valve device similar to the first embodiment is used as an example to demonstrate that the plunger 316 may be integrated with a connector part 316a forming a connection port of the valve 300. The connector part 316a may be of a female or male Luer type or may be attached to a tubing that may be flexible (not shown). The first fixation point 325 of the tubing 311 may be the same as explained in previous embodiments but the second fixation point is disclosed in this embodiment to be different than previously embodiments, where the plunger 316 may be attached to the flexible tubing 311 on the exterior of the flexible tubing 311 in a second fixation point 326.

An actuator 328 may be used to engage the plunger 316. The connection port 302b is shown here as a male Luer Lock connector but may also be of any Luer type or other suitable connectors or may be integrated with a tubing as shown in previous embodiments. It should be noted that this type of plunger, inlet, outlet and second fixation point may be combined with previous embodiments shown.

The closed distal end 19b of the plunger 16 used in the valve device 100 according to the first embodiment has been described as having a cross-sectional area exceeding the cross-sectional area of the central through-going opening 15 in the valve sealing member 8. Thus, the distal end 19b will effectively be able to close-off the opening 15 when abutting the valve sealing member 8. It is to be understood that a closing-off with remained function may be provided by the distal end 19b being slightly cone-shaped, thereby at least partly penetrating the through-going opening 15 in the valve sealing member 8.

The valve sealing member 208 used in the second embodiment of the valve device 200 shown best in Fig. 3D, may be provided with an optional recess 217 across its proximal and/or distal surface 208a, 208b. The recess 217 may be arranged to extend in the circumferential direction of the valve sealing member 208 and facilitate deflection of the valve sealing member 208 as seen in the longitudinal direction when subjected to an external force applied by a fluid flow or the plunger 216.

The fluid flow has been described as being arranged through the bore 20, 220,320 extending inside the plunger 16, 216,316. According to a non-disclosed embodiment, a fluid flow past the plunger 16, 216,316, may be allowed by the exterior wall of the plunger 16, 216,316, comprising a longitudinal groove and/or the inner wall 4a, 204a,304a of the passage 4, 204,304 in the housing 1,201,301 comprising a longitudinal groove. The fluid flow past the plunger 16, 216, 316 will thereby be allowed along the exterior wall of the plunger 16, 216, 316 instead of through a centrally arranged bore 20,220,320.

## Claims

1. Valve device (100; 200; 300) comprising
a housing (1; 201; 301) comprising a passage (4; 204; 304) extending between a proximal end (2a; 202a; 302a) and a distal end (2b; 202b; 302b) of the housing (1; 201; 301);
a plunger (16; 216; 316) arranged to extend at least partly inside the passage (4; 204; 304) of the housing (1, 201; 301);
a valve sealing member (8; 208; 308) arranged in the passage (4; 204; 304), the valve sealing member (8; 208; 308) together with the plunger (16; 216; 316) being configured to control a fluid flow through the passage (4; 204; 304);
a flexible tubing (11; 211; 311) having a distal end (10b; 210b; 310b)fixedly arranged to the housing (1; 201;301), thereby forming a first fixing portion (25; 225;325) of the flexible tubing (11; 211; 311);
wherein the plunger (16; 216; 316) having
a proximal end (19a; 219a; 319a) being arranged to the flexible tubing (11; 211; 311), thereby forming a second fixing portion (26; 226; 326) of the flexible tubing (11; 211; 311); and
a distal end (19b; 219b; 319b) configured to selectively act on the valve sealing member (8; 208; 308) for selectively setting the valve device (100; 200; 300) from an open state or a closed state to a forced open state;
wherein the valve device (100; 200; 300) allowing a fluid flow in a downstream direction in the open state from the proximal end (2a; 202a; 302a) of the housing (1; 201; 301) to the distal end (2b; 202b; 302b) of the housing (1; 201; 301), and restricting a fluid flow in an upstream direction in the closed state from the distal end (2b; 202b; 302b) of the housing (1; 201; 301) to the proximal end (2a; 202a; 302a) of the housing (1; 201; 301); and
wherein the valve device (100; 200; 300) upon being selectively set in the forced open state, a fluid flow in the upstream direction from the distal end (2b; 202b; 302b) of the housing (1; 201; 301) to the proximal end (2a; 202a; 302a) of the housing (1; 201; 301) is allowed; and
wherein the valve device (100; 200; 300) is arranged to be selectively set from an open state or closed state to the forced open state by affecting the length of the flexible tubing (11; 211; 311) as seen between its first fixing portion (25; 225; 325) and its second fixing portion (26; 226; 326), thereby displacing the plunger (16; 216; 316) relative the housing (1; 201; 301).

2. Valve device (100; 200; 300) according to any of the preceding claims, wherein the plunger (16, 216, 300) is relatively rigid as seen in its longitudinal direction in relation to the flexible tubing (11, 211, 311) that is flexible in its longitudinal direction.

3. Valve device (100; 200; 300) according to claims 1 or 2, wherein the first fixing portion (25; 225; 325) and the second fixing portion (26; 226; 326) of the flexible tubing (11; 211; 31) are separated by an intermediate portion extending along the longitudinal extension of the flexible tubing (11; 211, 311), and wherein the flexible tubing (11; 211; 311) along the intermediate portion is unfixed relative to the proximal part (2a,202a;302a) of the housing and the plunger (16;216;316) respectively.

4. Valve device (100; 200; 300) according to any of the preceding claims, wherein the plunger (16; 216; 316) comprises a through-going bore (20; 220; 320), an open proximal end (19a; 219a; 319a), and a radially extending opening (21; 221; 321) adjacent the distal end (19b; 219b; 319b), whereby the radially extending opening (21; 221; 321) allows fluid flow past the plunger (16; 216; 316) through said through-going bore (20; 220; 320).

5. Valve device (100; 200; 300) according to any of the preceding claims, wherein the valve device (100; 200; 300) is arranged to be selectively set to the forced open state by applying a force to an exterior side of the flexible tubing (11; 211; 311) and the housing (1; 201; 301) respectively, thereby causing a longitudinal length modification of the flexible tubing (11; 211; 311) as seen between its first fixing portion (25; 225; 325) and its second fixing portion (26; 226; 326).

6. Valve device (100;200) according to any of the preceding claims, wherein the valve device (100;200) further comprises an actuator (28;228), the actuator (28;228) being arranged on an exterior side of the flexible tubing (11;211) and on an exterior side of the housing (1;201), the actuator having a distal end and a proximal end, and the actuator (28;228) engaging the housing (1;201) by the distal end of the actuator and engaging on the exterior of the flexible tubing (11;211) by a proximal end of the actuator, whereby application of a force to the actuator (28;228) in a transverse direction causes a longitudinal length modification of the flexible tubing (11;211) between the first fixing position (25;225) and the second fixing position (26;226) thereby setting the valve device (100;200) to its forced open state.

7. Valve device (300) according to any of claims 1-5, wherein the valve device (300) further comprises an actuator (328), the actuator (328) being arranged on an exterior side of the flexible tubing (311) and on an exterior side of the housing (301), the actuator having a distal end and a proximal end, and the actuator (328) engaging the housing (301) by the distal end of the actuator and engaging the plunger (316) by the proximal end of the actuator, whereby application of a force to the actuator (328) in a transverse direction causes a longitudinal length modification of the flexible tubing (311) between the first fixing position (325) and the second fixing position (326) thereby setting the valve device (300) to its forced open state.

8. Valve device (100;300) according to any of claims 1-7, wherein the flexible valve sealing member (8;308) comprises a through-going opening (15;315), and wherein the distal end of the plunger (16;316) is arranged to abut the valve sealing member (8;308) when the valve device (100;300) is set to the closed state; whereby
a fluid flow in the downstream direction from the proximal end (2a;302a) of the housing (1;301) to the distal end (2b;302b) of the housing (1;301) is allowed by the fluid flow forcing the flexible valve sealing member (8;308) to unseat the distal end (19b;319b) of the plunger (16;316), thereby allowing a fluid flow through the through opening (15;315) in the flexible valve sealing member (8,308), and
a fluid flow in the upstream direction from the distal end (2b;302b) of the housing (1;301) to the proximal end (2a;302a) of the housing (1;301) is restricted by the fluid flow forcing the flexible valve sealing member (8;308) to sealingly close-off the through-going opening (15;315) in the flexible valve sealing member (8;308).

9. Valve device (200) according to any of claims 1-6, wherein the valve sealing member (208) is flexible and comprises a first portion (232) and a second portion (233), the second portion (233) encircling the first portion (232), the first portion (232) forming a homogenous sealing surface and the second portion comprising a through-going opening (215),
wherein when the valve device (200) is set in its forced open position, the plunger (216) is displaced relative to the housing (201) to such extent that the first portion (232) of the flexible sealing member (208) is deflected by the distal end (219b) of the plunger (216) acting thereon, thereby forming an interspace between the first portion (232) of the flexible sealing member (208) and the valve seat (231) to thereby allow a fluid flow in an upstream direction through the through-going opening (215) in the flexible sealing member (208).

10. Valve device (100; 200) according to any of claims 1-6, wherein the proximal end of the plunger (16; 216) comprises a transversely extending projection (27; 227), and wherein the proximal end (19a; 219a) of the plunger (16; 216) is fixedly arranged to the flexible tubing (11; 211) at the second fixing portion (26: 226) by the transversely extending projection (27; 227) engaging the inner proximal wall portion of the flexible tubing (11; 211).

11. Use of a valve device (100; 200; 300) according to any of claims 1-10 as a back check valve in an intravenous system.

12. Valve device (100; 200; 300) according to any of claims 1-10, wherein the exterior of the plunger (16; 216; 316) comprising a longitudinal groove to allow fluid flow past the exterior wall of the plunger (16; 216; 316).

13. Valve device (100; 200; 300) according to any of claims 1-10 or 12, wherein the inner wall (4a; 204a; 304a) of the passage (4; 204; 304) in the housing (1; 201; 301) comprising a longitudinal groove to allow fluid past the exterior wall of the plunger (16; 216; 316).

## Patentansprüche

1. Ventilvorrichtung (100, 200, 300) umfassend
ein Gehäuse (1; 201; 301), umfassend einen Durchgang (4; 204; 304), der sich zwischen einem proximalen Ende (2a; 202a; 302a) und einem distalen Ende (2b; 202b; 302b) des Gehäuses (1; 201; 301) erstreckt;
einen Kolben (16; 216; 316), der so angeordnet ist, dass er sich mindestens teilweise innerhalb des Durchgangs (4; 204; 304) des Gehäuses (1, 201; 301) erstreckt;
ein Ventildichtungselement (8; 208; 308), das in dem Durchgang (4; 204; 304) angeordnet ist, wobei das Ventildichtungselement (8; 208; 308) zusammen mit dem Kolben (16; 216; 316) konfiguriert ist, um einen Fluidstrom durch den Durchgang (4; 204; 304) zu steuern;
einen flexiblen Schlauch (11; 211; 311) mit einem distalen Ende (10b; 210b; 310b), das fest an dem Gehäuse (1; 201; 301) angeordnet ist, wodurch ein erster Befestigungsabschnitt (25; 225; 325) des flexiblen Schlauchs (11; 211; 311) gebildet wird;
wobei der Kolben (16; 216; 316) aufweist
ein proximales Ende (19a; 219a; 319a), das an dem flexiblen Schlauch (11; 211; 311) angeordnet ist, wodurch ein zweiter Befestigungsabschnitt (26; 226; 326) des flexiblen Schlauchs (11; 211; 311) gebildet wird; und
ein distales Ende (19b; 219b; 319b), das konfiguriert ist, um selektiv auf das Ventildichtungselement (8; 208; 308) einzuwirken, um die Ventilvorrichtung (100; 200; 300) selektiv von einem offenen Zustand oder einem geschlossenen Zustand in einen zwangsweise offenen Zustand einzustellen; wobei die Ventilvorrichtung (100; 200; 300) im offenen Zustand einen Fluidstrom in einer stromabwärtigen Richtung vom proximalen Ende (2a; 202a; 302a) des Gehäuses (1; 201; 301) zum distalen Ende (2b; 202b; 302b) des Gehäuses (1; 201; 301) ermöglicht und einen Fluidstrom in einer stromaufwärtigen Richtung im geschlossenen Zustand von dem distalen Ende (2b; 202b; 302b) des Gehäuses (1; 201; 301) zum proximalen Ende (2a; 202a; 302a) des Gehäuses (1; 201; 301) begrenzt; und
wobei die Ventilvorrichtung (100; 200; 300) nach dem selektiven Einstellen in den zwangsweise offenen Zustand ein Fluidstrom in der stromaufwärtigen Richtung von dem distalen Ende (2b; 202b; 302b) des Gehäuses (1; 201; 301) zu dem proximalen Ende (2a; 202a; 302a) des Gehäuses (1; 201; 301) ermöglicht wird; und
wobei die Ventilvorrichtung (100; 200; 300) angeordnet ist, um selektiv von einem offenen Zustand oder einem geschlossenen Zustand in den zwangsweise offenen Zustand eingestellt zu werden, indem die Länge des flexiblen Schlauchs (11; 211; 311) zwischen seinem ersten Befestigungsabschnitt (25; 225; 325) und seinem zweiten Befestigungsabschnitt (26; 226; 326) gesehen beeinflusst wird, wodurch der Kolben (16; 216; 316) relativ zum Gehäuse (1; 201; 301) verschoben wird.

2. Ventilvorrichtung (100; 200; 300) nach einem der vorhergehenden Ansprüche, wobei der Kolben (16, 216, 300) relativ starr ist, wenn er in seiner Längsrichtung in Bezug auf den flexiblen Schlauch (11, 211, 311), der in seiner Länge flexibel ist Längsrichtung, betrachtet wird.

3. Ventilvorrichtung (100; 200; 300) nach Anspruch 1 oder 2, wobei der erste Befestigungsabschnitt (25; 225; 325) und der zweite Befestigungsabschnitt (26; 226; 326) des flexiblen Schlauchs (11; 211; 31) durch einen Zwischenabschnitt getrennt sind, der sich entlang der Längsausdehnung des flexiblen Schlauchs (11; 211, 311) erstreckt, und wobei der flexible Schlauch (11; 211; 311) entlang des Zwischenabschnitts relativ zum proximalen Teil (2a, 202a; 302a) des Gehäuses bzw. des Kolbens (16; 216; 316) unbefestigt ist.

4. Ventilvorrichtung (100; 200; 300) nach einem der vorhergehenden Ansprüche, wobei der Kolben (16; 216; 316) eine Durchgangsbohrung (20; 220; 320), ein offenes proximales Ende (19a; 219a; 319a) und eine sich radial erstreckende Öffnung (21; 221; 321) benachbart zu dem distalen Ende (19b; 219b; 319b) umfasst, wobei die sich radial erstreckende Öffnung (21; 221; 321) einen Fluidstrom an dem Kolben (16; 216; 316) vorbei durch die Durchgangsbohrung (20; 220; 320) ermöglicht.

5. Ventilvorrichtung (100; 200; 300) nach einem der vorhergehenden Ansprüche, wobei die Ventilvorrichtung (100; 200; 300) angeordnet ist, um selektiv in den zwangsweise offenen Zustand eingestellt zu werden, indem eine Kraft an eine Außenseite des flexiblen Schlauchs (11; 211; 311) bzw. des Gehäuses (1; 201; 301) angelegt wird, wodurch eine Änderung der Länge in Längsrichtung des flexiblen Schlauchs (11; 211; 311) bewirkt wird, wie zwischen seinem ersten Befestigungsabschnitt (25; 225; 325) und seinem zweiten Befestigungsabschnitt (26; 226; 326) zu sehen ist.

6. Ventilvorrichtung (100; 200) nach einem der vorhergehenden Ansprüche, wobei die Ventilvorrichtung (100; 200) ferner einen Aktuator (28; 228) umfasst, wobei der Aktuator (28; 228) auf einer Außenseite des flexiblen Schlauchs (11; 211) und auf einer Außenseite des Gehäuses (1; 201) angeordnet ist, wobei der Aktuator ein distales Ende und ein proximales Ende aufweist und der Aktuator (28 ; 228) durch das distale Ende des Aktuators in das Gehäuse (1; 201) eingreift und auf der Außenseite des flexiblen Schlauchs (11; 211) durch ein proximales Ende des Aktuators eingreift, wodurch das Anlegen einer Kraft an den Aktuator (28; 228) in einer Querrichtung eine Veränderung der Länge in Längsrichtung des flexiblen Schlauchs (11; 211) zwischen der ersten Befestigungsposition (25; 225) und der zweiten Befestigungsposition (26; 226) bewirkt, wodurch die Ventilvorrichtung (100; 200) in ihren zwangsweise offenen Zustand eingestellt wird.

7. Ventilvorrichtung (300) nach einem der Ansprüche 1 bis 5, wobei die Ventilvorrichtung (300) ferner einen Aktuator (328) umfasst, wobei der Aktuator (328) an einer Außenseite des flexiblen Schlauchs (311) und an einer Außenseite des Gehäuses (301) angeordnet ist, wobei der Aktuator ein distales Ende und ein proximales Ende aufweist und der Aktuator (328) durch das distale Ende des Aktuators in das Gehäuse (301) eingreift und durch das proximale Ende in den Kolben (316) des Aktuators eingreift, wodurch das Anlegen einer Kraft an den Aktuator (328) in einer Querrichtung eine Änderung der Länge in Längsrichtung des flexiblen Schlauchs (311) zwischen der ersten Befestigungsposition (325) und der zweiten Befestigungsposition (326) bewirkt, wodurch die Ventilvorrichtung (300) in ihren zwangsweise offenen Zustand eingestellt wird.

8. Ventilvorrichtung (100; 300) nach einem der Ansprüche 1 bis 7, wobei das flexible Ventildichtungselement (8; 308) eine Durchgangsöffnung (15; 315) umfasst und wobei das distale Ende des Kolbens (16; 316) angeordnet ist, um an dem Ventildichtungselement (8; 308) anzuliegen, wenn die Ventilvorrichtung (100; 300) in den geschlossenen Zustand eingestellt ist; wodurch
ein Fluidstrom in Stromabwärtsrichtung vom proximalen Ende (2a; 302a) des Gehäuses (1; 301) zum distalen Ende (2b; 302b) des Gehäuses (1; 301) dadurch ermöglicht wird, dass der Fluidstrom das flexible Ventildichtungselement (8; 308) zwingt, das distale Ende (19b; 319b) des Kolbens (16; 316) zu lösen, wodurch ein Fluidstrom durch die Durchgangsöffnung (15; 315) in dem flexiblen Ventildichtungselement (8, 308) ermöglicht wird, und
ein Fluidstrom in der stromaufwärtigen Richtung vom distalen Ende (2b; 302b) des Gehäuses (1; 301) zum proximalen Ende (2a; 302a) des Gehäuses (1; 301) dadurch begrenzt wird, dass der Fluidstrom das flexible Ventildichtungselement (8; 308) zwingt, die Durchgangsöffnung (15; 315) in dem flexiblen Ventildichtungselement (8; 308) dichtend zu verschließen.

9. Ventilvorrichtung (200) nach einem der Ansprüche 1 bis 6, wobei das Ventildichtungselement (208) flexibel ist und einen ersten Abschnitt (232) und einen zweiten Abschnitt (233) umfasst, wobei der zweite Abschnitt (233) den ersten Abschnitt (232) umgibt, wobei der erste Abschnitt (232) eine homogene Dichtungsoberfläche bildet und der zweite Abschnitt eine Durchgangsöffnung (215) umfasst,
wobei, wenn die Ventilvorrichtung (200) in ihre zwangsweise offenen Position eingestellt wird, der Kolben (216) relativ zu dem Gehäuse (201) in einem solchen Ausmaß verschoben wird, dass der erste Abschnitt (232) des flexiblen Dichtungselements (208) durch das distale Ende (219b) des darauf einwirkenden Kolbens (216) abgelenkt wird, wodurch ein Zwischenraum zwischen dem ersten Abschnitt (232) des flexiblen Dichtungselements (208) und dem Ventilsitz (231) gebildet wird, um dadurch einen Fluidstrom in einer Stromaufwärtsrichtung durch die Durchgangsöffnung (215) in dem flexiblen Dichtungselement (208) zu ermöglichen.

10. Ventilvorrichtung (100; 200) nach einem der Ansprüche 1 bis 6, wobei das proximale Ende des Kolbens (16; 216) einen sich quer erstreckenden Vorsprung (27; 227) umfasst, und wobei das proximale Ende (19a; 219a) von dem Kolben (16; 216) an dem flexiblen Schlauch (11; 211) an dem zweiten Befestigungsabschnitt (26; 226) dadurch fest angeordnet ist, dass der sich quer erstreckende Vorsprung (27; 227) in den inneren proximalen Wandabschnitt des flexiblen Schlauchs (11; 211) eingreift.

11. Verwendung einer Ventilvorrichtung (100; 200; 300) nach einem der Ansprüche 1 bis 10 als Rückschlagventil in einem intravenösen System.

12. Ventilvorrichtung (100; 200; 300) nach einem der Ansprüche 1 bis 10, wobei das Äußere des Kolbens (16; 216; 316) eine Längsnut umfasst, um einen Fluidstrom an der Außenwand des Kolbens (16; 216; 316) zu ermöglichen.

13. Ventilvorrichtung (100; 200; 300) nach einem der Ansprüche 1 bis 10 oder 12, wobei die Innenwand (4a; 204a; 304a) des Durchgangs (4; 204; 304) in dem Gehäuse (1; 201; 301) eine Längsnut umfasst, um Fluid an der Außenwand des Kolbens (16; 216; 316) vorbeizulassen.

## Revendications

1. Dispositif de soupape (100 ; 200 ; 300) comprenant
un boîtier (1 ; 201 ; 301) comprenant un passage (4 ; 204 ; 304) s'étendant entre une extrémité proximale (2a, 202a ; 302a) et une extrémité distale (2b ; 202b ; 302b) du boîtier (1 ; 210 ; 301);
un piston (16, 216, 316) agencé pour s'étendre au moins partiellement à l'intérieur du passage (4 ; 204 ; 304) du boîtier (1 ; 210 ; 301) ;
un élément d'étanchéité de soupape (8 ; 208 ; 308) agencé dans le passage (4 ; 204 ; 304), l'élément d'étanchéité de soupape (8 ; 208 ; 308) ensemble avec le piston (16, 216, 316) étant configurés pour commander un écoulement de fluide à travers le passage (4 ; 204 ; 304) ;
un tube souple (11, 211, 311) ayant une extrémité distale (10b ; 210b ; 310b) agencé de manière fixe au boîtier (1 ; 210 ; 301), permettant ainsi de former une première partie de fixation (25 ; 225 ; 325) du tube souple (11, 211, 311) ;
dans lequel le piston (16, 216, 316) ayant
une extrémité proximale (19a ; 219a ; 319a) étant agencée sur le tube souple (11, 211, 311), permettant ainsi de former une seconde partie de fixation (26 ; 226 ; 326) du tube souple (11, 211, 311) ; et
une extrémité distale (19b ; 219b ; 319b) configurée pour agir de manière sélective sur l'élément d'étanchéité de soupape (8 ; 208 ; 308) pour régler de manière sélective le dispositif de soupape (100 ; 200 ; 300) à partir d'un état ouvert ou un état fermé vers un état ouvert forcé ;
dans lequel le dispositif de soupape (100 ; 200 ; 300) permettant un écoulement de fluide dans une direction avale dans l'état ouvert de l'extrémité proximale (2a, 202a ; 302a) du boîtier (1 ; 210 ; 301) à l'extrémité distale (2b ; 202b ; 302b) du boîtier (1 ; 210 ; 301), et contraignant un écoulement de fluide dans une direction amont dans l'état fermé à partir de l'extrémité distale (2b ; 202b ; 302b) du boîtier (1 ; 210 ; 301) vers l'extrémité proximale (2a, 202a ; 302a) du boîtier (1 ; 210 ; 301) ; et
dans lequel le dispositif de soupape (100 ; 200 ; 300) après avoir été réglé de manière sélective dans l'état ouvert forcé, un écoulement de fluide est permis dans la direction amont à partir de l'extrémité distale (2b ; 202b ; 302b) du boîtier (1 ; 210 ; 301) vers l'extrémité proximale (2a, 202a ; 302a) du boîtier (1 ; 210 ; 301) ; et
dans lequel le dispositif de soupape (100 ; 200 ; 300) est agencé pour être réglé de manière sélective depuis un état ouvert ou un état fermé vers l'état ouvert forcé en modifiant la longueur du tube souple (11, 211, 311) vu entre sa première partie de fixation (25 ; 225 ; 325) et sa seconde partie de fixation (26 ; 226 ; 326), permettant ainsi de déplacer le piston (16, 216, 316) par rapport au boîtier (1 ; 210 ; 301).

2. Dispositif de soupape (100 ; 200 ; 300) selon l'une quelconque des revendications précédentes, dans lequel le piston (16, 216, 300) est relativement rigide, dans sa direction longitudinale en ce qui concerne le tube souple (11, 211, 311) qui est souple dans sa direction longitudinale.

3. Dispositif de soupape (100 ; 200 ; 300) selon la revendication 1 ou la revendication 2, dans lequel la première partie de fixation (25 ; 225 ; 325) et la seconde partie de fixation (26 ; 226 ; 326) du tube souple (11 ; 211 ; 311) sont séparées par une partie intermédiaire s'étendant le long de l'extension longitudinale du tube souple (11 ; 211 ; 311), dans lequel le tube souple (11 ; 211 ; 311) le long de la partie intermédiaire n'est pas attaché par rapport à la partie proximale (2a, 202a ; 302a) du boîtier et du piston (16 ; 216 ; 316) respectivement.

4. Dispositif de soupape (100 ; 200 ; 300) selon l'une quelconque des revendications précédentes, dans lequel le piston (16, 216, 316) comprend un trou transversal traversant (20 ; 220 ; 320), une extrémité proximale ouverte (19a ; 219a ; 319a), et une ouverture s'étendant de manière radiale (21 ; 221 ; 321) adjacente à l'extrémité distale (19b ; 219b ; 319b), moyennant quoi l'ouverture s'étendant de manière radiale (21 ; 221 ; 321) permet au fluide de s'écouler le long du piston (16 ; 216 ; 316) par l'intermédiaire dudit trou transversal traversant (20 ; 220 ; 320).

5. Dispositif de soupape (100 ; 200 ; 300) selon l'une quelconque des revendications précédentes, dans lequel le dispositif de soupape (100 ; 200 ; 300) est agencé pour être réglé de manière sélective à l'état ouvert forcé en appliquant une force sur un côté extérieur du tube souple (11 ; 211 ; 311) et du boîtier (1 ; 201 ; 301) respectivement, provoquant ainsi une modification de longueur longitudinale du tube souple (11 ; 211 ; 311) vu entre sa première partie de fixation (25 ; 225 ; 325) et sa seconde partie de fixation (26 ; 226 ; 326).

6. Dispositif de soupape (100 ; 200 ; 300) selon l'une quelconque des revendications précédentes, dans lequel le dispositif de soupape (100 ; 200 ; 300) comprend en outre un actionneur (28 ; 228), l'actionneur (28 ; 228) étant agencé sur un côté extérieur du tube souple (11 ; 211) et sur un côté extérieur du boîtier (1 ; 201), l'actionneur ayant une extrémité distale et une extrémité proximale, et l'actionneur (28 ; 228) venant en prise avec le boîtier (1 ; 201) par l'extrémité distale de l'actionneur et venant en prise sur l'extérieur du tube souple (11 ; 211) par une extrémité proximale de l'actionneur, moyennant quoi l'application d'une force sur l'actionneur (28 ; 228) dans une direction transversale provoque une modification de longueur longitudinale du tube souple (11 ; 211) entre la première position de fixation (25 ; 225) et la seconde position de fixation (26 ; 226) permettant ainsi de mettre le dispositif de soupape (100 ; 200 ; 300) dans son état ouvert forcé.

7. Dispositif de soupape (100 ; 200 ; 300) selon l'une quelconque des revendications 1 à 5, dans lequel le dispositif de soupape (300) comprend en outre un actionneur (328), l'actionneur (328) étant agencé sur un côté extérieur du tube souple (311) et sur un côté extérieur du boîtier (301), l'actionneur ayant une extrémité distale et une extrémité proximale, et l'actionneur (328) venant en prise avec le boîtier (301) par l'extrémité distale de l'actionneur et venant en prise avec le piston (316) par l'extrémité proximale de l'actionneur, moyennant quoi l'application d'une force sur l'actionneur (328) dans une direction transversale provoque une modification de longueur longitudinale du tube souple (311) entre la première position de fixation (325) et la seconde position de fixation (326) permettant ainsi de mettre le dispositif de soupape (300) dans son état ouvert forcé.

8. Dispositif de soupape (100 ; 300) selon l'une quelconque des revendications 1 à 7, dans lequel l'élément d'étanchéité de soupape souple (8 ; 308) comprend une ouverture traversante (15 ; 315), et dans lequel l'extrémité distale du piston (16 ; 316) est agencée pour venir buter contre l'élément d'étanchéité de soupape (8 ; 308) lorsque le dispositif de soupape (100 ; 300) est mis dans son état fermé ; moyennant quoi
un écoulement fluide dans la direction avale de l'extrémité proximale (2a ; 302a) du boîtier (1 ; 301) à l'extrémité distale (2b ; 302b) du boîtier (1 ; 301) est permis par l'écoulement fluide forçant l'élément d'étanchéité de soupape souple (8 ; 308) à renverser l'extrémité distale (19b ; 319b) du piston (16 ; 316), permettant ainsi un écoulement fluide à travers l'ouverture traversante (15 ; 315) dans l'élément d'étanchéité de soupape souple (8 ; 308), et
un écoulement fluide dans la direction amont de l'extrémité distale (2b ; 302b) du boîtier (1 ; 301) à l'extrémité proximale (2a ; 202a) du boîtier (1 ; 301) est restreint par l'écoulement fluide forçant l'élément de étanchéité de soupape souple (8 ; 308) à obturer de manière étanche l'ouverture traversante (15 ; 315) dans l'élément d'étanchéité de soupape souple (8 ; 308).

9. Dispositif de soupape (200) selon l'une quelconque des revendications 1 à 6, dans lequel l'élément d'étanchéité de soupape (208) est souple et comprend une première partie (232) et une seconde partie (233), la seconde partie (233) entourant la première partie (232), la première partie (232) formant une surface d'étanchéité homogène et la seconde partie comprenant une ouverture traversante (215),
dans lequel lorsque le dispositif de soupape (200) est réglé dans sa position ouverte forcée, le piston (216) est déplacé par rapport au boîtier (201) à tel point que la première partie (232) de l'élément d'étanchéité souple (208) est déviée par l'extrémité distale (219b) du piston (216) agissant sur celle-ci, permettant ainsi de former un espace intermédiaire entre la première partie (232) de l'élément d'étanchéité souple (208) et le siège de soupape (231) pour ainsi permettre un écoulement de fluide dans une direction amont par l'intermédiaire de l'ouverture traversante (215) dans l'élément d'étanchéité souple (208).

10. Dispositif de soupape (100 ; 200) selon l'une quelconque des revendications 1 à 6, dans lequel l'extrémité proximale du piston (16 ; 216) comprend une saillie s'étendant de manière transversale (27 ; 227), et dans lequel extrémité proximale (19a ; 219a) du piston (16 ; 216) est agencée de manière fixe sur le tube souple (11 ; 211) au niveau de la deuxième partie de fixation (26 ; 226) au moyen de la saillie s'étendant de manière transversale (27 ; 227) venant en prise avec la partie de paroi proximale interne du tube souple (11 ; 211).

11. Dispositif de soupape (100 ; 200 ; 300) selon l'une quelconque des revendications 1 à 10 en tant que soupape anti-siphon dans un système intraveineux.

12. Dispositif de soupape (100 ; 200 ; 300) selon l'une quelconque des revendications 1 à 10, dans lequel l'extérieur du piston (16 ; 216 ; 316) comprenant une rainure longitudinale pour permettre un écoulement de fluide au-delà de la paroi externe du piston (16 ; 216 ; 316).

13. Dispositif de soupape (100 ; 200 ; 300) selon l'une quelconque des revendications 1 à 10 ou la revendications 12, dans lequel la paroi interne (4a ; 204a ; 304a) du passage (4 ; 204 ; 304) dans le boîtier (1 ; 201 ; 301) comprenant une rainure longitudinale pour permettre à un fluide de traverser la paroi externe du piston (16 ; 216 ; 316).
